# EUROPÄISCHE PATENTSCHRIFT

(11) **EP 1 013 246 B1**
(45) Veröffentlichungstag und Bekanntmachung des Hinweises auf die Patenterteilung: **01.10.2003**
(21) Anmeldenummer: 99811077.9
(22) Anmeldetag: 23.11.1999
(51) Int. Cl.: A61F 2/40

(54) **Glenoidprothese und Baukasten mit Glenoidprothesen**
Glenoid prosthesis and modular system with glenoid prostheses
Prothèse glénoidienne et système modulaire comportant des prothèses glénoidiennes

(30) Priorität: 22.12.1998 EP 98811255
(43) Veröffentlichungstag der Anmeldung: 28.06.2000
(73) Patentinhaber: Centerpulse Orthopedics Ltd., 6340 Baar (CH)
(72) Erfinder: Schoch, Roland, 6340 Baar (CH)
(74) Vertreter: Manitz, Finsterwald & Partner Gbr

(56) Entgegenhaltungen:
- EP-A- 0 581 667
- FR-A- 2 579 454
- US-A- 5 032 132

## Beschreibung

Die Erfindung handelt von einer Glenoidprothese mit einer Lagerschale deren Rückseite mehrere parallel zueinander angeordnete Verankerungszapfen aufweist.

In einem Prospekt: "Schulter-Endoprothese zementiert". Ausgabe 1998 der Sulzer Orthopedics Ltd., CH-6340 Baar, wird die Operationstechnik und das verwendete Material ausführlich beschrieben. Gezeigt ist eine Glenoidprothese mit vier Zapfen auf der Rückseite, die nach dem Fräsen und Bohren des natürlichen Glenoids einzementiert wird.

Aufgabe der vorliegenden Erfindung ist es, eine solche Glenoidprothese in einem grösseren Spektrum von Anwendungsfällen einsetzen zu können. Diese Aufgabe wird mit den Kennzeichen vom unabhängigen Anspruch 1 dadurch erfüllt, dass mindestens ein Verankerungszapfen ein Kupplungselement aufweist und dass mindestens eine Hülse mit einem passenden festsetzbaren Kupplungselement und einer äusseren Verankerungsstruktur vorgesehen ist, geeignet um wahlweise ein Einzementieren des Zapfens oder ein mechanisches Einschlagen des Zapfens zusammen mit der daran festgesetzten Hülse zu ermöglichen.

Diese Ausgestaltung der Glenoidprothese bringt mehrere Vorteile mit sich. Sie gestattet es, die Prothese wahlweise einzuzementieren oder nach dem Festsetzen von Hülsen mit Verankerungsstrukturen ohne Zement direkt im Knochen zu verankern. Beim Einzementieren hat die Kupplungsstruktur an den Zapfen den Vorteil, dass für den Zementmantel Vor- und Rücksprünge vorhanden sind und dass kein Überladen stattfindet, weil Zement in Vertiefungen zurückfliessen kann. Beim Einschlagen ohne Zement kann ein für das Einwachsen geeignetes Hülsenmaterial und eine für das Einschlagen geeignete Verankerungsstruktur auf der Hülse eingesetzt werden.

Die abhängigen Ansprüche 2 bis 12 stellen vorteilhafte Weiterbildungen der Erfindung dar. So ist es vorteilhaft, wenn die Zapfen als Kupplungselement ein Aussengewinde und die Hülsen als passendes Kupplungselement ein Innengewinde aufweisen. Ebenso sind Bajonettverschlüsse möglich. Ausserdem ist es vorteilhaft, wenn alle Zapfen unabhängig von der Grösse der Lagerschalen mit gleich grossen Gewindedurchmessern versehen sind, da nur ein zusätzliches Teil, die Hülse, in die Lagerhaltung aufgenommen werden muss. Im weiteren kann eine Lagerschale mit Zapfen aus einem Material gewählt werden, das gute Gleiteigenschaften hat, ohne dass die Verankerung darunter leiden muss. Eine Konstruktion aus Kunststoff in Form einer Lagerschale aus Polyethylen kombiniert mit metallischen Hülsen aus einer Titanlegierung bringt eine gute Verankerung. Diese kann verbessert werden, wenn in Längsrichtung messerartige Rippen über den Umfang der Hülse verteilt sind. Zum einen wird die Abstützfläche vergrössert und zum anderen das Knochenmaterial zum Einwachsen angeregt. Weiterhin kann das Einwachsen von Knochenmaterial verbessert werden, wenn die Rückseite der Lagerschale mit einem Metallgeflecht versehen ist, welches beispielsweise aus einer Titanlegierung besteht.

Ein weiterer Vorteil ergibt sich, wenn der Orthopäde erst nach dem Bohren der Bohrungen für die Verankerungszapfen entsprechend dem vorgefundenen Knochenzustand entscheiden kann, ob er zementieren oder ohne Zement einschlagen will. Dies wird dadurch erreicht, dass die Hülse eine so geringe Wandstärke hat, dass der Bohrungsdurchmesser, der einer angestrebten Dicke des Zementmantels beim Zementieren entspricht, gleichzeitig auch den angestrebten Bohrungsdurchmesser für das direkte Einschlagen von Hülsen mit einer Verankerungsstruktur bildet. D.h. die Verankerungsstruktur ist so ausgebildet relativ zum Zapfen, dass der Bohrungsdurchmesser kleiner als der Aussendurchmesser über die Verankerungsstruktur ist und in der Nähe vom Aussendurchmesser der eigentlichen Hülse liegt.

Im folgenden ist die Erfindung anhand von Ausführungsbeispielen beschrieben. Es zeigen:
- Fig. 1: Schematisch die Ansicht einer Lagerschale mit Gewindezapfen und einer aufschraubbaren Hülse mit Längsrippen;
- Fig. 2: schematisch eine Ansicht der Lagerschale der Fig. 1 von der Rückseite;
- Fig. 3: schematisch eine Seitenansicht der Lagerschale der Fig. 1;
- Fig. 4: schematisch die Rückansicht einer Lagerschale, die zusätzlich mit einem Metallgeflecht auf ihrer Rückseite versehen ist;
- Fig. 5: schematisch eine Seitenansicht der Lagerschale von Fig. 4;
- Fig. 6: schematisch eine Seitenansicht einer Hülse mit Verankerungsrippen und
- Fig. 7: schematisch eine Vorderansicht mit Blick auf die Gewindebohrung der Hülse in Fig. 6.

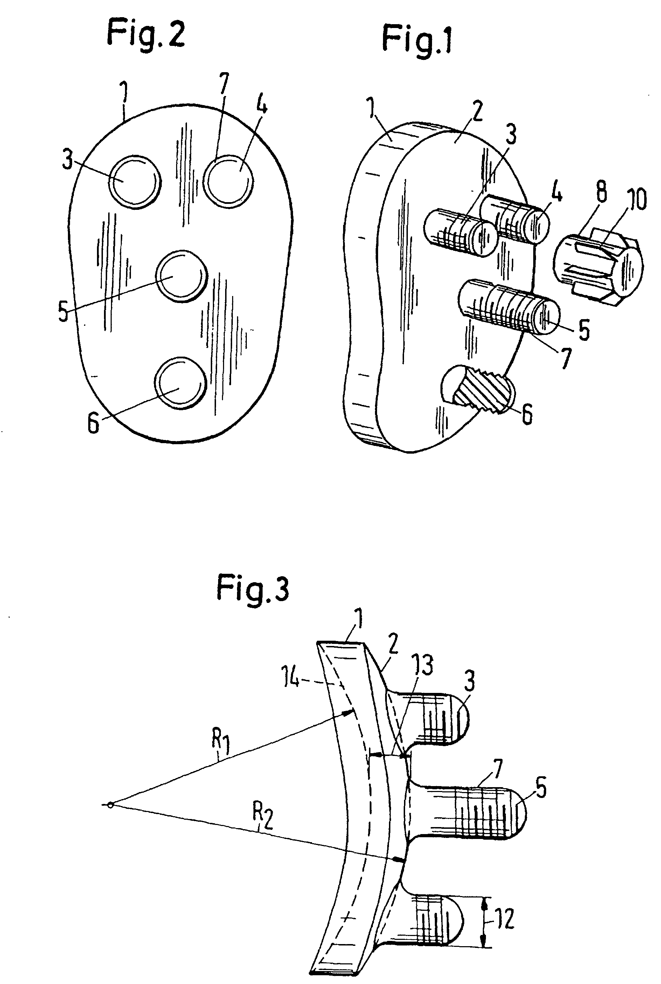

Die Figuren zeigen eine Glenoidprothese mit einer Lagerschale 1 deren Rückseite 2 mehrere parallel zueinander angeordnete Verankerungszapfen 3, 4, 5, 6 aufweist. Mindestens ein Verankerungszapfen weist ein Kupplungselement 7 auf und mindestens eine Hülse 8 mit einem passenden festsetzbaren Kupplungselement 9 und einer äusseren Verankerungsstruktur 10 ist vorgesehen, um wahlweise ein Einzementieren des Verankerungszapfens oder ein mechanisches Einschlagen des Zapfens zusammen mit der daran festgesetzten Hülse zu ermöglichen. In den Figuren 1, 2 und 3 ist eine Lagerschale 1 gezeigt, deren Lagerfläche 14 und deren Rückseite 2 Ausschnitte von Kugelflächen R₁, R₂ mit einem gemeinsamen Mittelpunkt sind. Das Material der Lagerschale 1 ist ein UHMW-Polyethylen und die Wandstärke 13, die dem Radiusunterschied R₂-R₁ darstellt, entspricht einem Betrag um 5,5 mm. Mit anderen Werkstoffen sind Wandstärken mit Werten zwischen 3 und 7 mm denkbar, wobei das Ziel darin besteht, unter Beibehaltung einer ausreichenden Lagerfunktion und Lagerstabilität eine möglichst geringe Wandstärke zu erreichen, da das natürliche Glenoid um etwa diesen Betrag eingefräst werden muss, wenn die Bänder nach dem Einsetzen der künstlichen Lagerschale gleich gespannt sein sollen.

Auf der Rückseite 2 der Lagerschale stehen vier Verankerungszapfen 3, 4, 5, 6 vor, welche jeweils mit einem Aussengewinde 7 versehen sind. Entsprechend den Befestigungsmöglichkeiten an einem natürlichen Glenoid sind Zapfen 3 und 4 horizontal angeordnet und Zapfen 5 und 6 vertikal angeordnet, wobei die Lagerschale zur Befestigung an einem aufrechtstehenden Menschen noch um 180° gedreht werden müsste. Die Gewindezapfen 3, 4, 5, 6 sind in diesem Fall einstückig mit der Lagerschale verbunden und parallel zueinander angeordnet, um sie gemeinsam in passend vorgebohrten Bohrungen einsetzen zu können. Der äussere Gewindedurchmesser 5 ist für alle Zapfen gleich. Entsprechend der Dicke eines natürlichen Glenoids steht der mittlere Zapfen 5 weiter vor für die Verankerung. Die Lagerfläche 14 der Lagerschale 1 ist mit einer für künstliche Gelenke ausreichende Oberflächengüte und Formgenauigkeit versehen.

Eine derartige Lagerschale kann mit ihren Zapfen direkt in mit Knochenzement gefüllte Bohrungen an einem natürlichen Glenoid eingepresst werden. Der über die Lagerschale hervorquellende Knochenzement wird abgestreift und die Lagerschale an Ort gehalten bis der Knochenzement ausgehärtet ist. Während dem Einpressen der Zapfen helfen die daran angebrachten Gewinde beim Entlüften von eingeschlossenen Luftblasen, da der Knochenzement nicht sofort bis in den Gewindegrund benetzt. Nach dem Aushärten bilden die Gewindeflächen klar definierte Anlageflächen zur Kraftübertragung in der Richtung der Zapfen.

Sollte ein Operateur nach dem Bohren der Aufnahmelöcher im natürlichen Glenoid zur Auffassung kommen, dass die Knochensubstanz für eine direkte Verankerung stabil genug ist, dann kann er die zum Bausatz gehörenden metallischen Hülsen 8 auf die Gewindezapfen 3, 4, 5, 6 aufschrauben und ohne Knochenzement mit der Lagerschale einschlagen. Die Wandstärke der Hülse 8 ist gering ausgeführt, damit die Bohrungen im natürlichen Glenoid (hier nicht gezeigt) wahlweise für eine direkte Verankerung der Hülsen 8 oder für eine Verankerung der Zapfen mit Knochenzement verwendet werden können. Die Hülsen 8 sind auf ihrer Vorderseite geschlossen und weisen messerartige Längsrippen über den Umfang verteilt auf, welche sich beim Einschlagen in das Knochengewebe eingraben. Die Primärbefestigung entsteht in diesem Fall durch die Reibungskräfte an den Rippen 10 und Hülsen 8. Als Metall für die Hülsen 8 sind Reintitan oder körperverträgliche Titanlegierungen vorteilhaft.

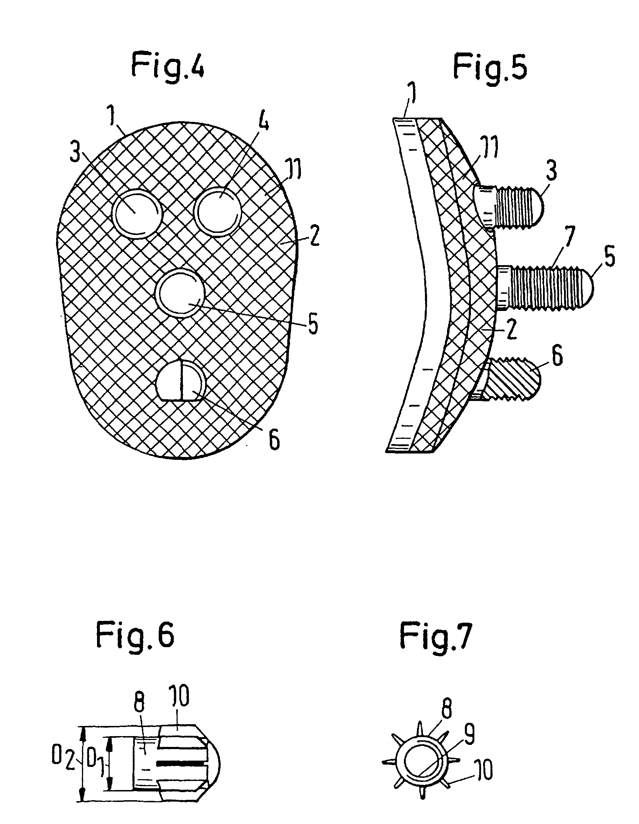

Im Beispiel von Figur 4 und 5 ist eine Lagerschale 1 aus Polyethylen auf ihrer Rückseite 2 mit einem Metallgeflecht 11 versehen. Ein an seiner Aussenkante verrundetes Metallgeflecht "mesh" wird erwärmt und auf der Rückseite 2 der Lagerschale 1 angepresst. Das Metallgeflecht 11 dringt teilweise in das Polyethylen ein und einzelne Drähte des Geflechts werden von aufgeschmolzenem Polyethylen umschlossen, um eine Verankerung über die ganze Fläche zu erreichen. Wenn die Lagerzapfen bei dieser Prozedur nicht verletzt werden sollen, besteht eine Möglichkeit darin das Metallgeflecht 11 mit Aussparungen zu versehen und die Zapfen 3, 4, 5, 6 aus Polyethylen später an der Rückseite 2 durch Reibschweissen an der Lagerschale 1 zu befestigen. Eine andere Möglichkeit besteht darin, den Zapfen für die Dauer des Zusammenfügens von Lagerschale 1 und Metallgeflecht 11 eine Schutzhülse aus einem schlechten Wärmeleiter aufzusetzen. Eine solche Lagerschale hat zusätzlich zu den wahlweise einsetzbaren Hülsen 8 den Vorteil, dass auf der Rückseite 2 eine Struktur angebracht ist, die sowohl für eine Befestigung mit Knochenzement als auch für ein direktes Einwachsen im Knochen geeignet ist.

Die Figuren 6 und 7 zeigen eine Hülse 8 in Seitenansicht und von hinten. Die messerartig ausgeformten Längsrippen 10 sind gleichmässig über den Umfang verteilt. Für die Lagerschalen sind Bohrlehren vorgesehen. Die mit Bohrlehren im Knochen erzeugten Bohrungen haben einen Durchmesser D₂>d≥0,9D₁, wobei der Durchmesser D₂ über die Rippen 10 gemessen ist und der Durchmesser D₁ dem Aussendurchmesser des Hülsenkörpers entspricht. Dieser Durchmesser d erzeugt gleichzeitig eine beim Einzementieren angestrebte Schichtdicke von Knochenzement zwischen Bohrung und Zapfen 3, 4, 5, 6. Die Vorderseite der Hülse 8 ist patronenförmig verrundet.

## Patentansprüche

1. Glenoidprothese mit einer Lagerschale (1) deren Rückseite (2) mehrere parallel zueinander angeordnete Verankerungszapfen (3, 4, 5, 6) aufweist, wobei mindestens ein Verankerungszapfen ein Kupplungselement (7) aufweist, **dadurch gekennzeichnet, dass** die Glenoidprothese mindestens eine Hülse (8) mit einem zum Kupplungselement des Verankerungszapfens passenden festsetzbaren Kupplungselement (9) und einer äusseren Verankerungsstruktur (10) umfaßt geeignet um wahlweise ein Einzementieren des Zapfens oder ein mechanisches Einschlagen des Zapfens zusammen mit der daran festgesetzten Hülse (8) zu ermöglichen.

2. Prothese nach Anspruch 1, **dadurch gekennzeichnet, dass** das Kupplungselement (7) eines Verankerungszapfens aus einem Aussengewinde besteht und dass das festsetzbare Kupplungselement (9) der Hülse (8) aus einem Innengewinde besteht.

3. Prothese nach Anspruch 1 oder 2, **dadurch gekennzeichnet, dass** alle Verankerungszapfen (3, 4, 5, 6) mit Aussengewinden (7) versehen sind.

4. Prothese nach einem der Ansprüche 1 bis 3, **dadurch gekennzeichnet, dass** die Lagerschale (1) und die Zapfen (3, 4, 5, 6) aus Kunststoff, beispielsweise aus Polyethylen,bestehen.

5. Prothese nach einem der Ansprüche 1 bis 4, **dadurch gekennzeichnet, dass** die Hülsen (8) an ihrer Stirnseite geschlossen sind und aus Metall, beispielsweise aus einer Titanlegierung bestehen.

6. Prothese nach einem der Ansprüche 1 bis 5, **dadurch gekennzeichnet, dass** die Hülsen (8) über den Umfang verteilte Längsrippen (10) aufweisen.

7. Prothese nach Anspruch 6, **dadurch gekennzeichnet, dass** die Längsrippen (10) messerartig ausgebildet sind und/oder Unterbrüche aufweisen.

8. Prothese nach einem der Ansprüche 1 bis 7, **dadurch gekennzeichnet, dass** die Rückseite (2) der Lagerschale (1) mit Ausnahme der Zapfen (3, 4, 5, 6) ein metallisches Geflecht (11) aufweist.

9. Prothese nach Anspruch 8 im Rückbezug auf Anspruch 4, **dadurch gekennzeichnet, dass** die Zapfen (3, 4, 5, 6) durch Reibschweissen an der Schale (1) befestigt sind.

10. Prothese nach einem der Ansprüche 1 bis 9, **dadurch gekennzeichnet, dass** die Lagerfläche (14) der Lagerschale (1) und die Rückseite (2) der Lagerschale Ausschnitte von Kugelflächen (R₁, R₂) sind und dass die Lagerschale (1) eine Wandstärke (13) zwischen 3 mm und 7 mm aufweist.

11. Baukasten mit Glenoidprothesen nach einem der Ansprüche 2 bis 10, **dadurch gekennzeichnet, dass** Lagerschalen (1) mit unterschiedlichen Aussenabmessungen vorhanden sind, und dass die Gewindedurchmesser (12) der Zapfen (3, 4, 5, 6) für alle Lagerschalen (1) gleich gross sind, um mit einer Hülsengrösse auszukommen.

12. Baukasten mit Glenoidprothesen nach Anspruch 11 im Rückbezug auf Anspruch 6, **dadurch gekennzeichnet, dass** für die Lagerschalen (1) Bohrlehren vorgesehen sind, deren Führungsdurchmesser für Bohrer mit Durchmesser D₂>d≥0,9D₁ versehen ist, wobei der Durchmesser D₂ dem Durchmesser der Hülsen (8) über die Rippen (10) gemessen entspricht und der Durchmesser D₁ dem Durchmesser über den Hülsenkörper entspricht.

## Claims

1. Glenoid prosthesis comprising a bearing shell (1), the reverse side (2) of which has a plurality of anchoring pins (3, 4, 5, 6) which are arranged parallel to one another, wherein at least one anchoring pin has a coupling element (7), **characterised in that** the glenoid prosthesis has at least one sleeve (8) with a securable coupling element (9) matching the coupling element of the anchoring pin and includes an outer anchoring structure (10) which is suitable in order to selectively enable a cementing in of the pin or a mechanical hammering in of the pin together with the sleeve (8) which is fixed to it.

2. Prosthesis in accordance with claim 1, **characterised in that** the coupling element (7) of an anchoring pin consists of an outer thread; and **in that** the securable coupling element (9) of the sleeve (8) consists of an inner thread.

3. Prosthesis in accordance with claim 1 or claim 2, **characterised in that** all anchoring pins (3, 4, 5, 6) are provided with outer threads (7).

4. Prosthesis in accordance with any one of the claims 1 to 3, **characterised in that** the bearing shell (1) and the pins (3, 4, 5, 6) consist of plastic, for example of polyethylene.

5. Prosthesis in accordance with any one of the claims 1 to 4, **characterised in that** the sleeves (8) are closed at their front ends and consist of metal, for example of a titanium alloy.

6. Prosthesis in accordance with any one of the claims 1 to 5, **characterised in that** the sleeves (8) have longitudinal ribs (10) which are distributed over the periphery.

7. Prosthesis in accordance with claim 6, **characterised in that** the longitudinal ribs (10) are designed in the manner of knives and/or have interruptions.

8. Prosthesis in accordance with any one of the claims 1 to 7, **characterised in that** with the exception of the pins (3, 4, 5, 6) the reverse side (2) of the bearing shell (1) has a metallic mesh (11).

9. Prosthesis in accordance with claim 8 when dependent on claim 4, **characterised in that** the pins (3, 4, 5, 6) are fastened to the shell (1) through friction welding.

10. Prosthesis in accordance with any one of the claims 1 to 9, **characterised in that** the bearing surface (14) of the bearing shell (1) and the reverse side (2) of the bearing shell are sections of spherical surfaces (R₁, R₂); and **in that** the bearing shell (1) has a wall thickness (13) between 3 mm and 7 mm.

11. Modular system comprising glenoid prostheses in accordance with any one of the claims 2 to 10, **characterised in that** bearing shells (1) with different outer dimensions are present; and **in that** the thread diameters (12) of the pins (3, 4, 5, 6) are equally large for all bearing shells (1) in order to be able to manage with one sleeve size.

12. Modular system comprising glenoid prostheses in accordance with claim 11 when dependent on claim 6, **characterised in that** bore gauges are provided for the bearing shells (1), the guide diameter of which is furnished for drills with diameter D₂ > d ≥ 0.9D₁, with the diameter D₂ corresponding to the diameter of the sleeves (8) which is measured across the ribs (10) and the diameter D₁ corresponding to the diameter across the sleeve body.

## Revendications

1. Prothèse glénoïdienne avec une coquille de support (1) dont la face arrière (2) comprend plusieurs tenons d'ancrage (3, 4, 5, 6) disposés parallèlement les uns par rapport aux autres, moyennant quoi au moins un tenon d'ancrage comprend un élément de raccordement (7), **caractérisé en ce que** la prothèse glénoïdienne comprend au moins une gaine (8) avec un élément de raccordement (9) pouvant être fixé et adapté à l'élément de raccordement du tenon d'ancrage et une structure d'ancrage externe (10) afin de permettre au choix une implémentation du tenon avec du ciment ou un impactage mécanique du tenon dans la gaine solidement fixée (8).

2. Prothèse selon la revendication 1, **caractérisée en ce que** l'élément de raccordement (7) d'un tenon d'ancrage se compose d'un filetage externe et **en ce que** l'élément de raccordement (9) de la gaine (8) pouvant être fixé se compose d'un filetage interne.

3. Prothèse selon la revendication 1 ou 2, **caractérisée en ce que** tous les tenons d'ancrage (3, 4, 5, 6) sont dotés de filetages externes (7).

4. Prothèse selon l'une des revendications 1 à 3, **caractérisée en ce que** la coquille de support (1) et les tenons (3, 4, 5, 6) se composent de matière plastique, par exemple du polyéthylène.

5. Prothèse selon l'une des revendications 1 à 4, **caractérisée en ce que** les gaines (8) sont fermées sur leur face avant et se composent de métal, par exemple d'un alliage de titane.

6. Prothèse selon l'une des revendications 1 à 5, **caractérisée en ce que** les gaines (8) comportent des rainures longitudinales (10) réparties sur leur périphérie.

7. Prothèse selon la revendication 6, **caractérisée en ce que** les rainures longitudinales (10) sont réalisées sous forme de lames et / ou comportent des interruptions.

8. Prothèse selon l'une des revendications 1 à 7, **caractérisée en ce que** la face arrière (2) de la coquille de support (1) comporte, à l'exception des tenons (3, 4, 5, 6) un treillis métallique (11).

9. Prothèse selon la revendication 8 se rapportant à la revendication 4, **caractérisée en ce que** les tenons (3, 4, 5, 6) sont fixés à la coquille (1) au moyen d'un soudage par friction.

10. Prothèse selon l'une des revendications 1 à 9, **caractérisée en ce que** la surface de support (14) de la coquille de support (1) et la face arrière (2) de la coquille de support sont des sections de surfaces sphériques (R₁, R₂) et que la coquille de support (1) a une épaisseur de paroi (13) se situant entre 3 mm et 7 mm.

11. Système modulaire avec des prothèses glénoïdiennes selon l'une des revendications 2 à 10, **caractérisé en ce que** les coquilles de support (1) ont des dimensions externes différentes et que les diamètres des filetages (12) des tenons (3, 4, 5, 6) ont une taille identique pour toutes les coquilles de support (1), afin de s'adapter à une taille de gaine.

12. Système modulaire avec des prothèses glénoïdiennes selon la revendication 11, se rapportant à la revendication 6, **caractérisé en ce qu'**il est prévu pour les coquilles de support (1) des gabarits de forage dont le diamètre de guidage pour les forages comporte des diamètres D₂ > d ≥ 0,9 D₁, moyennant quoi le diamètre D₂ correspond au diamètre des gaines (8) au-dessus des rainures (10) et le diamètre D₁ correspond au diamètre au-dessus des corps des gaines.
